# FASCICULE DE BREVET EUROPEEN

(11) **EP 3 128 983 B1**
(45) Date de publication et mention de la délivrance du brevet: **24.02.2021**
(21) Numéro de dépôt: 15713902.3
(22) Date de dépôt: 07.04.2015
(51) Int. Cl.: A61F 13/62, A44B 18/00, B32B 5/26, D04H 13/00

(54) **PROCEDE POUR IMPARTIR DU DEVELOPPE AGRIPPANT A UN STRATIFIE ET STRATIFIE OBTENU PAR LE PROCEDE.**
VERFAHREN ZUR BEREITSTELLUNG EINES LAMINATS MIT EINEM KLETTVERSCHLUSSVOLUMEN UND RESULTIERENDES LAMINAT
METHOD FOR PROVIDING A LAMINATE WITH A HOOK AND LOOP FASTENING VOLUME, AND RESULTING LAMINATE

(30) Priorité: 08.04.2014 FR 1400848; 16.07.2014 FR 1401591
(43) Date de publication de la demande: 15.02.2017
(73) Titulaire: Aplix, 44850 Le Cellier (FR)
(72) Inventeur: MOINARD, Nathalie, 44980 Sainte Luce sur Loire (FR); MARCHE, Thierry, 44450 La Chapelle Basse Mer (FR)
(74) Mandataire: Eidelsberg, Olivier Nathan
(86) Numéro de dépôt international: PCT/EP2015/057441
(87) Numéro de publication internationale: WO 2015/155150

(56) Documents cités:
- EP-A1- 1 900 512
- WO-A1-97/19808
- WO-A1-2008/130807
- US-A- 5 326 612
- US-A1- 2003 077 430
- US-A1- 2006 003 658

## Description

### DOMAINE TECHNIQUE

La présente invention se rapporte à un procédé pour impartir du développé agrippant à un stratifié comportant au moins une couche formant support, par exemple une couche de non tissé, et au moins une couche de non tissé à boucles fixée sur la au moins une couche inférieure formant support. La présente invention se rapporte également à un dispositif pour impartir du développé agrippant à un stratifié de ce genre ainsi qu'à un stratifié de ce genre.

Un stratifié comportant au moins une couche formant support, par exemple une couche de non tissé, et au moins une couche de non tissé à boucles, par exemple un non tissé cardé consolidé par calandrage, est notamment utilisé pour former la partie boucles d'un autoagrippant à crochets et boucles, notamment pour des couches culottes et notamment pour former une bande disposée dans la partie avant de la couche culotte, au niveau de la ceinture en position centrale, bande classiquement appelée « bande confort » ou « landing zone », pour permettre la fermeture de la ceinture de la couche culotte par l'accroche de crochets issus de pattes disposées dans des parties d'extrémités latérales de la partie arrière de la couche culotte.

Dans la présente invention, on entend par développé agrippant, le volume disponible dans le non tissé à boucles dans lequel des crochets peuvent pénétrer pour réaliser l'accrochage.

Les zones agrippantes des non tissés sont réalisées notamment par l'assemblage d'une nappe de fibres sur un support non tissé de type SMS. Les fibres sont indépendantes lors de la fabrication et il faut une grande quantité de fibres pour réaliser les volumes agrippants et principalement seules les fibres à la surface opposée au support peuvent coopérer avec les crochets. Il est donc nécessaire d'avoir une grande quantité de fibres et un poids important de produit pour avoir une coopération satisfaisante avec les crochets pour répondre aux attentes de l'utilisateur en matière de performances. La grande quantité de fibres implique de plus une mauvaise lisibilité de l'impression réalisée sur l'une des faces du support SMS.

Il existe en outre une contradiction entre le poids de filaments utilisés et la capacité de pénétration des crochets dans la zone agrippante. Plus le poids utilisé de fibres à iso volume est grand, plus la pénétration des crochets est difficile.

En général, une nappe formant bande confort est en forme de stratifié et comporte un élément support, par exemple un non-tissé SMS, sur lequel peuvent notamment être imprimés des motifs décoratifs et d'autre part un non tissé à boucles, par exemple un non-tissé cardé calandré, qui est fixé sur la partie supérieure de l'élément support.

De préférence, le non tissé cardé doit permettre à un utilisateur de voir à travers les motifs imprimés sur l'élément support.

D'autre part, on souhaite que la coopération entre les boucles agrippantes formées par l'élément textile et les crochets doit être optimale. Pour ce faire, il est nécessaire que les crochets puissent s'introduire ou s'engager facilement dans les boucles agrippantes. En outre, une bonne résistance au cisaillement des boucles lorsqu'elles sont sollicitées par un crochet est préférable ainsi qu'une bonne résistance à l'ouverture en pelage. On souhaite aussi avoir une bonne sensation d'accroché ressentie par l'utilisateur lors de l'ouverture lui donnant confiance dans la fermeture sans à-coup avec un effort mesuré. Cette appréciation est usuellement mesurée par le calcul de l'énergie au pelage. On souhaite aussi obtenir une bonne qualité sensorielle entraînant une sensation de douceur nécessitant un toucher doux et un aspect textile, ainsi qu'une optimisation du poids de la bande confort et donc à un coût réduit du produit.

Il en résulte des exigences contradictoires avec le souhait que les motifs imprimés puissent être bien vus à travers l'élément à boucles qui font que les bandes confort actuelles, notamment celles formées à partir de deux non-tissés respectivement un SMS et un cardé, soit ne présentent pas une bonne résistance au pelage, que ce soit dans la réalité ou dans le ressenti de l'utilisateur, soit ne permettent pas à l'utilisateur de bien voir les motifs imprimés sur la couche support.

On connaît de WO2008/130807 un stratifié comportant au moins un non tissé support et un non tissé cardé à boucle fixé sur l'élément support. Ce stratifié est fabriqué par calandrage créant des zones de liaison multiples. Il manque de développé agrippant.

### OBJET ET RESUME DE L'INVENTION

La présente invention vise à surmonter les inconvénients de l'art antérieur en proposant un procédé pour impartir du développé agrippant à un stratifié du genre défini ci-dessus, notamment destiné à former une bande confort, sans pour autant diminuer la visibilité de motifs imprimés sur la couche support.

Suivant l'invention, un procédé pour impartir du développé agrippant est tel que défini à la revendication 9.

On obtient ainsi de manière très simple un stratifié qui tout en permettant une excellente visibilité de motifs éventuellement imprimés sur l'élément support, présente plus de développé agrippant et également un capacité d'accroche plus grande pour des crochets.

Suivant un mode de réalisation préféré de l'invention, on règle les première et deuxième vitesses de sorte que le rapport de la première vitesse sur la deuxième vitesse (ou coefficient de suralimentation) soit supérieur à 1,1, en particulier supérieur à 1,2, et notamment compris entre 1,2 et 1,6, de préférence entre 1,3 et 1,5.

De préférence, au moins l'un des deux rouleaux est chauffé et la fixation des deux nappes est réalisée par calandrage.

De US 2006/0003658, on connaît un stratifié suivant le préambule de la revendication 1.

De EP 1 900 512, il est connu un stratifié élastique comportant un élément support élastique et un non-tissé cardé laminés l'un à l'autre avec interposition d'une couche de colle, puis étirés ensemble pour activer le stratifié, c'est-à-dire lui donner de l'élasticité.

De WO 2008/130807, il est connu un stratifié suivant le préambule de la revendication 1. A la figure 2A, il est représenté pour ce stratifié la courbe donnant la force d'étirement nécessaire en fonction de la déformation jusqu'à la rupture R. Cette courbe, partant de l'origine (Force nulle pour déformation égale à 0) ne comporte aucun point d'inflexion.

De US 5 858 518, il est connu un stratifié comportant au moins un élément support qui n'est pas élastique et au moins un non-tissé à boucles fixé par calandrage sur l'élément support, le stratifié étant obtenu par un procédé dans lequel on déroule une nappe de non-tissé à boucles et une nappe formant un élément support pour les faire passer dans un interstice formé entre deux rouleaux supérieur et inférieur pour réaliser une fixation des deux éléments l'un avec l'autre.

La présente invention se rapporte également à un stratifié tel que défini à la revendication 1.

Suivant un autre mode de réalisation, l'élément support est constitué d'un film plastique, notamment en matière thermoplastique.

De préférence, l'élément support n'est pas élastique. Dans le cas où l'élément support est élastique, la courbe donnant la force en fonction de la déformation (allongement) qu'il convient de considérer est la courbe de la première déformation qui commence à partir d'une déformation nulle.

### DESCRIPTION SUCCINCTE DES DESSINS

On décrit maintenant, uniquement à titre d'exemples et d'illustration, des modes de réalisation de l'invention, en se reportant aux dessins, dans lesquels,
- La figure 1: représente un stratifié suivant un mode de réalisation de l'invention ;
- La figure 2: représente la courbe donnant la force d'étirement en fonction de la déformation du stratifié de la figure 1 ;
- la figure 2A: représente la courbe donnant la force en fonction de la déformation pour un stratifié de l'art antérieur, dans lequel aucune survitesse n'a été créée lors du calandrage ;
- la figure 3: est un schéma de principe vu de côté d'un dispositif mettant en œuvre le procédé suivant l'invention ; et
- la figure 4: représente les performances caractéristiques d'un non tissé en fonction de la position en % du point d'inflexion.

### MODES DE REALISATION PREFERES

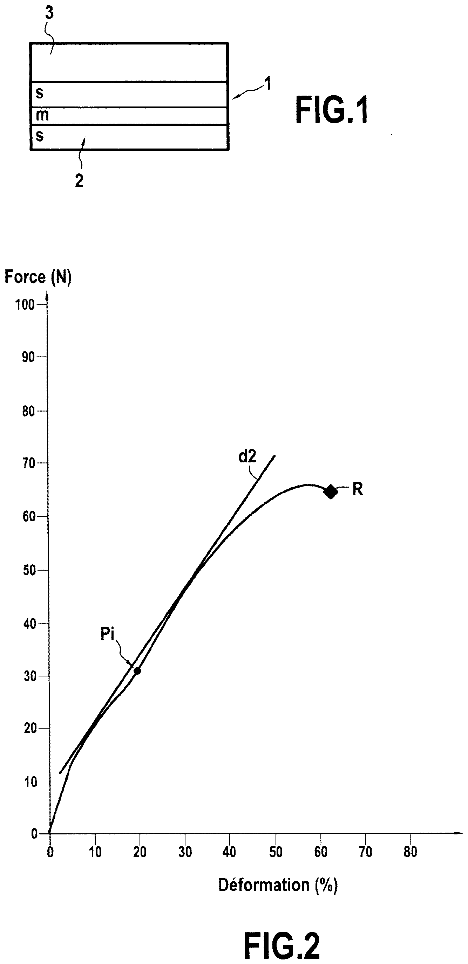

A la figure 1, il est représenté un stratifié 1 suivant l'invention, comportant un élément support 2 constitué d'une couche de non tissé SMS (c'est à dire constitué de trois couches superposées de non tissés Spunbond-Meltblown-Spunbond) et un élément 3 à boucles constitué d'un non tissé cardé consolidé par calandrage.

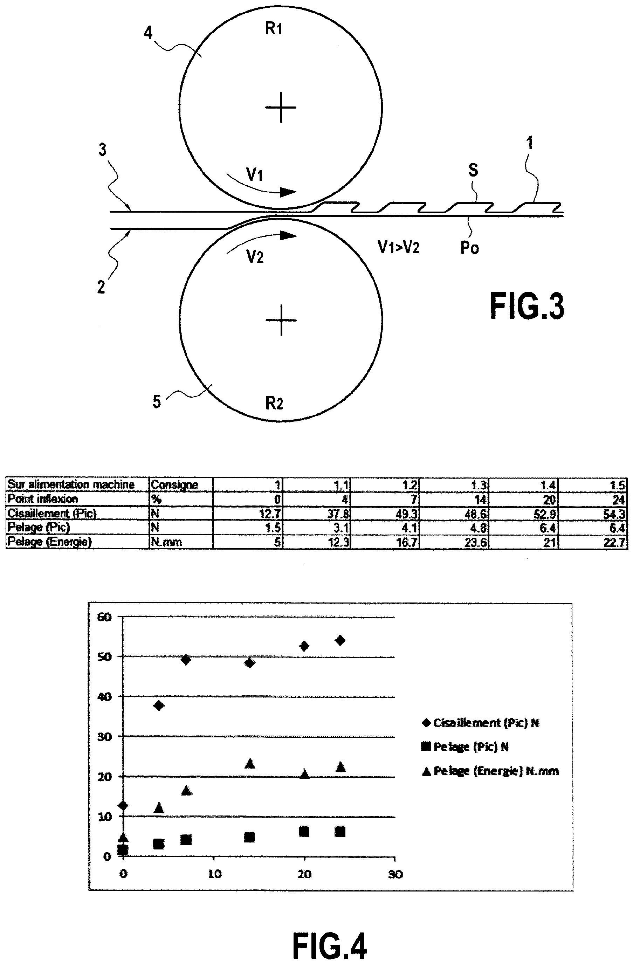

Des motifs ont été imprimés sur la face supérieure de l'élément support 2, puis les deux éléments 2 et 3 ont été envoyés ensemble entre deux rouleaux 4 et 5 pour leur fixation mutuelle par calandrage, comme représenté à la figure 3. Selon une variante, on peut réaliser l'impression sur la face inférieure de l'élément support 2.

Dans l'interstice formé entre les deux rouleaux supérieur 4 et inférieur 5, les deux éléments 2 et 3 sont pressés l'un contre l'autre par les surfaces extérieures respectives en regard l'une de l'autre des deux rouleaux. L'un ou les deux rouleaux est/sont chauffé(s) et l'un des deux rouleaux comporte un motif de gravure.

Le stratifié 1 de la figure 1 est celui obtenu en sortie du dispositif représenté à la figure 3.

En outre, les vitesses respectives des deux rouleaux inférieur et supérieur au niveau du contact avec le stratifié passant dans l'interstice sont orientées mutuellement parallèles, dans la direction de déroulement des deux éléments, notamment la direction machine ou MD. Cependant, la valeur de la vitesse V1 du rouleau supérieur au niveau du contact avec l'élément textile à boucle dans l'interstice est supérieure à celle de la vitesse V2 du rouleau inférieur, c'est à dire du rouleau en contact avec l'élément support. Pour obtenir le stratifié de la figure 1, on a réglé le rapport V1/V2 à la figure 3 pour qu'il soit égal à 1,4.

Comme on le voit à la figure 3, l'élément à boucle comporte en succession des zones de liaison, notamment par calandrage, et des zones formant les boucles. Les zones formant les boucles ont une forme asymétrique. En particulier, leur sommet respectif ou le milieu S de leur partie de sommet (dans le cas d'un sommet aplati comme à la figure 3) est décalé, notamment dans la direction MD, par rapport au point P0 central équidistant des deux zones de liaison successives entre lesquelles s'étend la zone formant boucle respective. En d'autres termes, le segment de droite SP0 n'est pas perpendiculaire au plan du non tissé support.

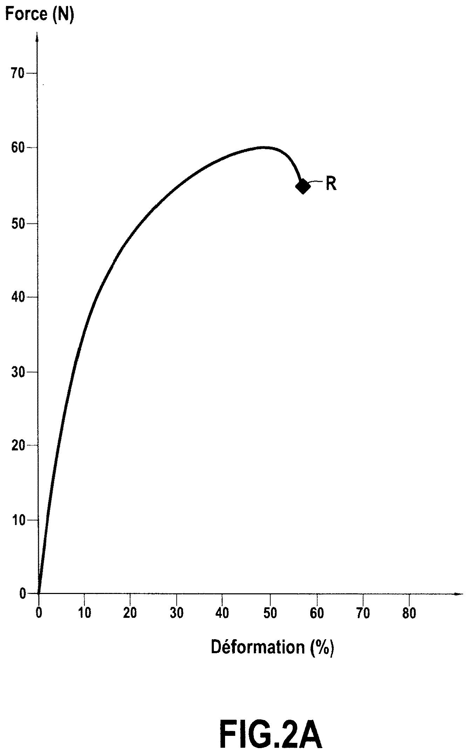

A la figure 2, on a représenté la courbe donnant la force d'allongement du stratifié 1 dans la direction MD en fonction de l'allongement. Pour réaliser cette courbe, on utilise le test de l'élongation à la rupture dans le sens MD, qui consiste à disposer un échantillon de longueur supérieure à 100mm et de 50mm de large du stratifié 1 obtenu en sortie du dispositif représenté à la figure 3 entre deux mâchoires (distance inter mâchoires égale à 100mm) d'un dynamomètre (vitesse de traction de 50mm/min) .

Comme on peut le voir, la courbe est croissante à partir de l'allongement 0% jusqu'à un point R aux alentours de 60% correspondant à la force maximale d'allongement ou encore à la force à la rupture. Au delà du point R, la courbe décroît, notamment avec une forte pente, très supérieure en valeur absolue à la pente du tronçon entre 0 et R.

Pour mesurer le point d'inflexion, on peut utiliser toute méthode classique, par exemple graphiquement par la méthode des tangentes (d2) ou par modélisation polynomiale, notamment de rang 4, de la courbe.

Sur le tronçon entre 0 et R, la courbe comporte un point d'inflexion Pi, aux alentours de 20%.Le point d'inflexion Pi est préférentiellement supérieur à 4%, notamment compris entre 7% et 30%, plus préférentiellement compris entre 7 et 20%.

En fonction de la suralimentation, le développé agrippant va croître, augmentant ainsi les performances, notamment en cisaillement et en pelage, du stratifié, notamment de la bande confort, comme le montre la figure 4.

Comme on le voit à la figure 2A, lorsque aucune survitesse n'est impartie à l'un des rouleaux (V₁V₂ = 1), la courbe ne comporte pas le point d'inflexion. Le stratifié résultant ne possède pas de développé agrippant.

### Méthode de pelage

Pour mesurer les performances en pelage en fonction de la suralimentation, on utilise une mesure de la résistance à l'ouverture à 180° d'un couple crochet/bande confort assemblés. Une bande de crochets de 13mm de large et de 25,4mm de long assemblée sur un support papier de 80g/m² et de largeur 25,4mm est pressée sur un échantillon de bande confort de dimension 50mmx50mm à l'aide d'un rouleau simple de 2kg, les orientations relatives des produits étant identiques à celles utilisées sur la couche culotte. Une traction de 1kg est ensuite réalisé pendant 10 secondes au support du crochet de manière à simuler la fermeture d'une couche culotte, notamment d'une couche culotte ayant des oreilles élastiques. Le papier supportant le crochet est ensuite inséré dans la mâchoire supérieure mobile d'un bâti de traction du type Synergie 200H de MTS System équipé d'une cellule dynamométrique de 100N et la bande confort est insérée dans la mâchoire inférieure. La distance entre les deux mâchoires est de 50mm. Pour mesurer la force d'ouverture, la partie haute du bâti effectue ensuite une translation de bas en haut à une vitesse de 305mm/min. On relève alors la valeur de la force maximale fournie par la machine ainsi qu'éventuellement la valeur d'énergie correspondant à l'aire sous la surface de la courbe test pris sur les 13 premiers mm de course du bâti de traction.

### Méthode de cisaillement

Pour mesurer les performances en cisaillement en fonction de la suralimentation, on prend un échantillon de 50mmx50mm de bande confort que l'on colle sur une plaque rigide, par exemple métallique, avec un adhésif double face.

Une bande de crochets de 13 mm de large et de 25,4mm de long assemblée sur un support papier de 250g/m² est engagée par l'opérateur sur la bande confort en respectant les orientations relatives des produits sur la couche et une pression est exercée pendant 3s par l'opérateur avec le pouce.

Une traction de 1kg est ensuite réalisée pendant 5 secondes au support du crochet de manière à simuler la fermeture d'une couche culotte, notamment d'une couche culotte ayant des oreilles élastiques.

La plaque métallique supportant la bande confort est insérée dans la mâchoire supérieure mobile d'un bâti de traction du type DY 30 de MTS System équipé d'une cellule dynamométrique de 100N.
Le papier supportant le crochet est ensuite inséré dans la mâchoire inférieure fixe.
Le mouvement du bâti sera dans le même sens que la traction de 1kg. La distance entre les deux mâchoires est de 76mm. Pour mesurer la force d'ouverture, la partie haute du bâti effectue ensuite une translation de bas en haut à une vitesse constante de 305mm/min. Le test est effectué jusqu'à ce que la boucle et le crochet soient totalement désengagés. On relève alors la valeur de force maximale sur la courbe obtenue.

Dans la présente invention, on entend par non tissé un produit obtenu à l'issue de la formation d'une nappe de fibres ou de filaments qui ont été consolidés. La consolidation peut être mécanique, chimique ou thermique et se traduit par la présence de liaison entre les fibres et/ou les filaments. Cette consolidation peut être directe, c'est-à-dire faite directement entre les fibres et/ou filaments par soudure, ou elle peut être indirecte, c'est-à-dire par l'intermédiaire d'une couche intermédiaire entre les fibres et/ou les filaments, par exemple une couche de colle ou une couche de liant. Le terme non-tissé se rapporte à une structure en forme de ruban ou nappe de fibres ou filaments qui sont entrelacées d'une manière non uniforme, irrégulière ou au hasard. Un non-tissé peut avoir une structure de couche unique ou une structure à couches multiples. Un non-tissé peut également être réuni à un autre matériau tel qu'un film pour former un stratifié. Un non-tissé peut être réalisé à partir de différents matériaux synthétiques et/ou naturels. Les matériaux naturels à titre d'exemple sont des fibres de cellulose, telles que coton, jute, pâte à papier, lin et analogue et peuvent également inclure des fibres de cellulose re-traitées, telles que la rayonne ou la viscose. Les fibres naturelles pour un matériau non-tissé peuvent être préparées en utilisant divers procédés tels que le cardage. Des matériaux synthétiques à titre d'exemple comportent, mais sans s'y limiter, des polymères thermoplastiques synthétiques, qui sont connus pour former des fibres qui incluent, sans s'y limiter, les polyoléfines, par exemple le polyéthylène, polypropylène, polybutylène et analogue ; le polyamide, par exemple le polyamide 6, polyamide 6.6, polyamide 10, polyamide 12 et analogue ; des polyesters, par exemple des polyéthylènes téraphthalates, des polybutylènes téréphtalates, des acides polylactiques et analogues, des polycarbonates, des polystyrènes, des élastomères thermoplastiques, des vinyles polymères, des polyuréthanes et des mélanges et des co-polymères de ces derniers.

De manière générale, les fibres et les filaments diffèrent principalement par leur longueur et par leur procédé de fabrication.

On entend par filaments les éléments unitaires, de très grandes longueurs vis à vis du diamètre dans lequel s'inscrit leur section, extrudés de manière continue pour former directement une nappe de non tissé qui peut être ensuite consolidée par thermo-liage ou tout autre moyen pour permettre l'atteinte des performances souhaitées et/ou leur transport. De préférence, les filaments présentent une longueur supérieure à 120mm.

On entend par fibre le terme générique pour désigner une matière textile ou un élément de matière textile de longueur réduite, inférieure à la longueur des filaments, et susceptible d'être filée et/ou utilisée dans la réalisation de non tissés. On distingue deux types de fibres, les fibres courtes formées de matière discontinue de faible longueur inférieure à 70mm (préférentiellement de 25mm à 60 mm) et les fibres longues formées de manière discontinue de grande longueur supérieure à 70 mm (préférentiellement de 80 mm à 120 mm).

A la différence des filaments qui sont consolidés directement après avoir été extrudés, les fibres sont communément orientées et organisées en nappe lors d'une étape de cardage bien connue de l'homme du métier. Cette nappe peut être ensuite consolidée par thermo-liage ou tout autre moyen pour permettre l'atteinte des performances souhaitées et/ou leur transport. Selon l'invention, on peut avantageusement utiliser un non tissé à boucles comprenant des fibres à haute élongation.

Par fibres à haute élongation, on entend des fibres qui présentent une élongation maximale avant la rupture supérieure à 250% d'élongation, c'est à dire des fibres qui peuvent s'étirer d'au moins 2,5 fois leur longueur au repos et avant étirement.

Plus particulièrement, les fibres à haute élongation présentent une élongation maximale avant la rupture supérieure à 300% d'élongation, ou encore présentent une élongation maximale avant la rupture comprise entre 300% et 600% d'élongation, plus particulièrement comprise entre 450% et 500% d'élongation.

Selon un mode de réalisation, le non tissé à boucles comprend des fibres à haute élongation, notamment au moins 50% de fibres à haute élongation.
Suivant la présente invention, on entend par boucle un filament et/ou une fibre qui comprend deux extrémités chacune solidaire du support en un point respectif du support ou en un même point du support. Une boucle peut également être formée de plusieurs filaments ou fibres solidarisés entre eux et dont au moins deux d'entre eux sont solidarisés au support, en un point ou en deux points respectifs distincts. Les boucles peuvent présenter ici une forme particulière dissymétrique.

On entend par non tissé à boucles un non tissé formant des boucles disponible pour un crochet après liaison au support.

Dans une variante, on pourrait envisager d'activer le non tissé à boucles avant de le fixer sur l'élément support.

De préférence au moins un élément support est un non tissé notamment un SM (Spunbond-Meltblown), un SMS (Spunbond-Meltblown-Spunbond) ou un SMMS (Spunbond-Meltblown-Meltblown-Spunbond), SSMMS (Spunbond-Spunbond-Meltblown-Meltblown-Spunbond) et analogue.

Suivant l'invention, on entend par élastique, un élément qui a, suivant le test ci-dessous, une rémanence ou SET' inférieure à 15%, de préférence inférieure à 10%, plus préférablement inférieure à 5% pour un étirement de 100% de sa dimension initiale. Suivant l'invention, on entend par non élastique un élément qui n'entre pas dans la définition d'un élément élastique ci dessus. De même, un élément qui rompt avant d'atteindre un allongement de 100% de sa dimension initiale doit être considéré comme un non élastique.

On peut aussi par exemple mesurer l'élasticité d'un stratifié en en déterminant la rémanence par le test suivant :
On conditionne l'échantillon dans une atmosphère normale, telle que définie dans la norme ASTDM 5170, température de 23°C ± 2°C et humidité relative de 50% ± 5 %.

On utilise comme appareillage un dynamomètre conforme à la norme EN 10002, notamment le Synergie 200, une colonne disponible auprès de la société MTS Systems Corp, U.S.A., conjointement avec un logiciel d'utilisation TESTWORKS 4.12.

On prépare l'échantillon en découpant au cutter ou aux ciseaux le produit élastique (par exemple, le stratifié de l'invention) en un échantillon de 50 mm de largeur dans le sens MD (direction de la machine, perpendiculairement au plan de la figure 1) et une longueur dans le sens CD (direction transversale, direction horizontale à la figure 1) de 120 mm.

Les paramètres sont sélectionnés comme suit :
Distance inter mâchoires : 100 mm
Vitesse machine : 508 mm/mn
Nombre de cycles : 1
Allongement du produit : 100% à vitesse constante

On étire le produit à 100% par déplacement vertical de la mâchoire supérieure, la mâchoire inférieure étant fixe, puis on le maintient dans la position pendant 30 secondes, puis on revient à la position initiale à vitesse constante où on le laisse 60 secondes (fin du cycle), puis on l'étire de nouveau jusqu'à la rupture du produit.

On obtient alors la courbe donnant la force d'étirement en fonction de l'allongement en %, celle-ci présentant une hystérésis qui permet de déterminer le Set par la formule de calcul suivante :
SET= L1-L0
Avec :
L0 : Point d'intersection avec l'axe des X (Allongement en %) lors du démarrage du test, soit le début du cycle.
L1 : Point d'intersection avec l'axe des X (Allongement en %) lors du démarrage du deuxième allongement après le retour à une force nulle et l'attente de 60 secondes.

## Revendications

1. Stratifié (1) comportant au moins un élément support (2) qui n'est pas élastique, par exemple un non-tissé, notamment un SMS, et au moins un non-tissé (3) à boucles, notamment un cardé, fixé par calandrage sur l'élément support (2), le stratifié pouvant être obtenu par un procédé qui comprend les étapes dans lesquelles on déroule une nappe de non-tissé à boucles et une nappe formant un élément support pour les faire passer dans un interstice formé entre deux rouleaux supérieur et inférieur pour réaliser une fixation des deux éléments l'un avec l'autre, la face du rouleau supérieur en contact avec le non-tissé à boucles ayant, au niveau de l'interstice, une première vitesse orientée dans le sens de déroulement du stratifié, ou sens machine ou MD, la face du rouleau inférieur en contact avec l'élément support ayant, au niveau de l'interstice, une deuxième vitesse également orientée dans le sens de déroulement du stratifié, ou sens machine ou MD et on règle les première et deuxième vitesses de sorte que la première vitesse soit supérieure à la deuxième vitesse et étant **caractérisé en ce que** la courbe donnant la force nécessaire pour étirer le stratifié suivant un axe donné, par exemple l'axe MD ou l'axe CD du stratifié, en fonction de l'allongement, comporte un tronçon dans lequel, à partir d'une valeur nulle de l'allongement, la force est croissante de zéro jusqu'à une force maximale, correspondant notamment à la rupture du stratifié, et la courbe comporte un point (Pi) d'inflexion sur ledit un tronçon.

2. Stratifié suivant la revendication 1, **caractérisé en ce que** le point (Pi) d'inflexion est supérieur à 4%, notamment est compris entre 7% et 30%, plus préférablement compris entre 7% et 20%.

3. Stratifié suivant la revendication 1 ou 2, **caractérisé en ce que** le au moins un élément (2) support est un non-tissé, notamment un SM (Spunbond-Meltblown), un SMS (Spunbond-Meltblown-Spunbond) ou un SMMS (Spunbond-Meltblown-Meltblown-Spunbond) ou un SSMMS (Spunbond-Spunbond-Meltblown-Meltblown-Spunbond).

4. Stratifié suivant l'une des revendications 1 à 3, **caractérisé en ce que** le non-tissé (3) à boucles est un cardé, notamment consolidé par calandrage.

5. Stratifié suivant la revendication 4, **caractérisé en ce que** le cardé (3) comprend des fibres à haute élongation.

6. Stratifié suivant l'une des revendications précédentes, **caractérisé en ce que** l'élément à boucle comporte en succession des zones de liaison et des zones formant les boucles, les zones formant les boucles ayant une forme asymétrique.

7. Stratifié suivant la revendication 6, **caractérisé en ce que** le sommet d'une zone formant boucle ou le milieu S de sa partie de sommet, dans le cas d'un sommet aplati, est décalé, notamment dans la direction MD, par rapport au point P0 central équidistant des deux zones de liaison successives entre lesquelles s'étend la zone formant boucle.

8. Procédé pour impartir du développé agrippant à un stratifié (1) suivant l'une des revendications précédentes, notamment destiné à former une bande confort, comportant au moins un élément support (2) textile, notamment au moins un non-tissé, par exemple SMS, et au moins un non-tissé (3) à boucles, notamment un non-tissé cardé, fixé sur la face supérieure de l'élément support, qui comprend les étapes dans lesquelles :
on déroule une nappe de non-tissé à boucles et une nappe formant un élément support pour les faire passer dans un interstice formé entre deux rouleaux supérieur et inférieur pour réaliser une fixation des deux éléments l'un avec l'autre, la face du rouleau supérieur en contact avec le non-tissé à boucles ayant, au niveau de l'interstice, une première vitesse orientée dans le sens de déroulement du stratifié, ou sens machine ou MD, la face du rouleau inférieur en contact avec l'élément support ayant, au niveau de l'interstice, une deuxième vitesse également orientée dans le sens de déroulement du stratifié, ou sens machine ou MD, est **caractérisé en ce que**:
on règle les première et deuxième vitesses de sorte que la première vitesse soit supérieure à la deuxième vitesse.

9. Procédé suivant la revendication 8, **caractérisé en ce que** l'on règle les première et deuxième vitesses de sorte que le rapport de la première vitesse sur la deuxième vitesse (ou coefficient de suralimentation) soit compris entre 1,2 et 1,6, de préférence entre 1,3 et 1,5.

10. Procédé suivant la revendication 8 ou 9, **caractérisé en ce qu'**au moins l'un des deux rouleaux est chauffé et la fixation des deux nappes est réalisée par calandrage.

## Patentansprüche

1. Schichtstoff (1), der mindestens ein Stützelement (2), das nicht elastisch ist, beispielsweise einen Vliesstoff, insbesondere einen SMS, und mindestens einen Flauschvliesstoff (3), insbesondere einen kardierten Vliesstoff, der durch Kalandrieren auf dem Stützelement (2) befestigt ist, umfasst, wobei der Schichtstoff durch ein Verfahren erhalten werden kann, das die Schritte beinhaltet, während derer eine Lage eines Flauschvliesstoffs und eine Lage, die ein Stützelement bildet, abgewickelt werden, um sie durch einen Spalt, der zwischen zwei Walzen, einer oberen und einer unteren, gebildet wird, zu führen, um eine Befestigung der zwei Elemente aneinander zu erzielen, wobei die Fläche der oberen Walze in Kontakt mit dem Flauschvliesstoff im Bereich des Spalts eine erste Geschwindigkeit aufweist, die in die Abwickelrichtung des Schichtstoffs bzw. die Maschinen- oder MD-Richtung weist, wobei die Fläche der unteren Walze in Kontakt mit dem Stützelement im Bereich des Spalts eine zweite Geschwindigkeit aufweist, die ebenfalls in die Abwickelrichtung des Schichtstoffs bzw. die Maschinen- oder MD-Richtung weist, und die erste und die zweite Geschwindigkeit so gesteuert werden, dass die erste Geschwindigkeit größer als die zweite Geschwindigkeit ist, und **dadurch gekennzeichnet, dass** die Kurve, die die notwendige Kraft bereitstellt, um den Schichtstoff gemäß einer gegebenen Achse, beispielsweise der Achse MD oder der Achse CD des Schichtstoffs, in Abhängigkeit von der Ausdehnung zu strecken, einen Abschnitt umfasst, in dem die Kraft ausgehend von einem Ausdehnungswert Null von Null bis auf eine Maximalkraft ansteigt, die insbesondere dem Bruch des Schichtstoffs entspricht, und die Kurve einen Wendepunkt (Pi) auf dem einen Abschnitt umfasst.

2. Schichtstoff nach Anspruch 1, **dadurch gekennzeichnet, dass** der Wendepunkt (Pi) größer als 4 % ist, insbesondere zwischen 7 % und 30 % liegt, bevorzugter zwischen 7 % und 20 % liegt.

3. Schichtstoff nach Anspruch 1 oder 2, **dadurch gekennzeichnet, dass** das mindestens eine Stützelement (2) ein Vliesstoff, insbesondere ein SM (Spunbond-Meltblown), ein SMS (Spunbond-Meltblown-Spunbond) oder ein SMMS (Spunbond-Meltblown-Meltblown-Spunbond) oder ein SSMMS (Spunbond-Spunbond-Meltblown-Meltblown-Spunbond) ist.

4. Schichtstoff nach einem der Ansprüche 1 bis 3, **dadurch gekennzeichnet, dass** der Flauschvliesstoff (3) ein kardierter Vliesstoff ist, der insbesondere durch Kalandrieren verfestigt wird.

5. Schichtstoff nach Anspruch 4, **dadurch gekennzeichnet, dass** der kardierte Vliesstoff (3) Fasern mit hoher Dehnung beinhaltet.

6. Schichtstoff nach einem der vorhergehenden Ansprüche, **dadurch gekennzeichnet, dass** das Flauschelement aufeinanderfolgend Verbindungsbereiche und flauschbildende Bereiche umfasst, wobei die flauschbildenden Bereiche eine asymmetrische Form aufweisen.

7. Schichtstoff nach Anspruch 6, **dadurch gekennzeichnet, dass** die Spitze eines flauschbildenden Bereichs oder, im Fall einer abgeflachten Spitze, die Mitte S seines Spitzenabschnitts im Verhältnis zu dem Mittelpunkt P0, der sich von den zwei aufeinanderfolgenden Verbindungsbereichen, zwischen denen sich der flauschbildende Bereich erstreckt, gleich weit entfernt befindet, insbesondere in der MD-Richtung versetzt ist.

8. Verfahren zum Aufbringen eines Klettverschlussvolumens auf einen Schichtstoff (1) nach einem der vorhergehenden Ansprüche, das insbesondere dazu bestimmt ist, ein Komfortband zu bilden, umfassend mindestens ein textiles Stützelement (2), insbesondere mindestens einen Vliesstoff, beispielsweise einen SMS, und mindestens einen Flauschvliesstoff (3), insbesondere einen kardierten Vliesstoff, der auf der oberen Fläche des Stützelements befestigt ist, das die Schritte beinhaltet, während derer:
eine Lage eines Flauschvliesstoffs und eine Lage, die ein Stützelement bildet, abgewickelt werden, um sie durch einen Spalt, der zwischen zwei Walzen, einer oberen und einer unteren, gebildet wird, zu führen, um eine Befestigung der zwei Elemente aneinander zu erzielen, wobei die Fläche der oberen Walze in Kontakt mit dem Flauschvliesstoff im Bereich des Spalts eine erste Geschwindigkeit aufweist, die in die Abwickelrichtung des Schichtstoffs bzw. die Maschinen- oder MD-Richtung weist, wobei die Fläche der unteren Walze in Kontakt mit dem Stützelement im Bereich des Spalts eine zweite Geschwindigkeit aufweist, die ebenfalls in die Abwickelrichtung des Schichtstoffs bzw. die Maschinen- oder MD-Richtung weist, und **dadurch gekennzeichnet, dass**:
die erste und die zweite Geschwindigkeit so gesteuert werden, dass die erste Geschwindigkeit größer als die zweite Geschwindigkeit ist.

9. Verfahren nach Anspruch 8, **dadurch gekennzeichnet, dass** die erste und die zweite Geschwindigkeit so gesteuert werden, dass das Verhältnis der ersten Geschwindigkeit zu der zweiten Geschwindigkeit (bzw. der Voreilkoeffizient) zwischen 1,2 und 1,6, vorzugsweise zwischen 1,3 und 1,5 liegt.

10. Verfahren nach Anspruch 8 oder 9, **dadurch gekennzeichnet, dass** mindestens eine der zwei Walzen beheizt wird und die Befestigung der zwei Lagen durch Kalandrieren erfolgt.

## Claims

1. A laminate (1) comprising at least one support element (2) which is not elastic, for example a non-woven fabric, in particular an SMS, and at least one non-woven fabric (3) with loops, in particular a carded non-woven fabric, secured by calendering to the support element (2), the laminate being obtainable by a method comprising the steps in which a strip of non-woven fabric with loops and a strip forming a support element are unrolled in order to pass them into a gap formed between two upper and lower rollers in order to secure the two elements to one another, the face of the upper roller in contact with the non-woven fabric with loops having, at the gap, a first speed orientated in the direction of unrolling the laminate, or in the direction of the machine or MD, the face of the lower roller in contact with the support element having, at the gap, a second speed likewise orientated in the direction of unrolling the laminate, or in the direction of the machine or MD, and the first and the second speeds are adjusted so that the first speed is greater than the second speed and being **characterized in that** the curve representing the force required to stretch the laminate along a given axis, for example the MD axis or the CD axis of the laminate, as a function of elongation, comprises a segment in which, starting from a zero elongation value, the force increases from zero up to a maximum force, corresponding in particular to the rupture of the laminate, and the curve comprises an inflection point (Pi) on said segment.

2. The laminate according to Claim 1, **characterized in that** the inflection point (Pi) is greater than 4%, in particular is between 7% and 30%, more preferably between 7 and 20%.

3. The laminate according to Claim 1 or 2, **characterized in that** the at least one support element (2) is a non-woven fabric, in particular an SM (Spunbond-Meltblown), an SMS (Spunbond-Meltblown-Spunbond), or an SMMS (Spunbond-Meltblown-Meltblown-Spunbond), SSMMS (Spunbond-Spunbond-Meltblown-Meltblown-Spu nbond).

4. The laminate according to any of Claims 1 to 3, **characterized in that** the non-woven fabric with loops (3) is a carded non-woven fabric, in particular consolidated by calendering.

5. The laminate according to Claim 4, **characterized in that** the carded non-woven fabric (3) comprises fibers with high elongation.

6. The laminate according to any of the preceding claims, **characterized in that** the element with loops comprises, in succession, connection zones and zones forming the loops, the zones forming the loops being asymmetrical in form.

7. The laminate according to Claim 6, **characterized in that** the top of a zone forming loops or the middle S of its top part, in case of a flattened top, is offset, in particular in the MD direction, with respect to the central point P0 equidistant from the two successive connection zones between which the zone forming loops extends.

8. A method for providing a laminate (1) with a gripping capacity, in particular according to any of the preceding claims, in particular intended to form a landing zone, comprising at least one textile support element (2), in particular at least one non-woven fabric, for example SMS, and at least one non-woven fabric (3) with loops, in particular a carded non-woven fabric, secured to the upper face of the support element, which comprises the steps in which:
a strip of non-woven fabric with loops and a strip forming a support element are unrolled in order to pass them into a gap formed between two upper and lower rollers in order to secure the two elements to one another, the face of the upper roller in contact with the non-woven fabric with loops having, at the gap, a first speed orientated in the direction of unrolling the laminate, or in the direction of the machine or MD, the face of the lower roller in contact with the support element having, at the gap, a second speed likewise orientated in the direction of unrolling the laminate, or in the direction of the machine or MD, is **characterized in that**:
the first and the second speeds are adjusted so that the first speed is greater than the second speed.

9. The method according to Claim 8, **characterized in that** the first and the second speeds are adjusted so that the ratio of the first speed to the second speed (or overfeeding coefficient) is between 1.2 and 1.6, preferably between 1.3 and 1.5.

10. The method according to Claim 8 or 9, **characterized in that** at least one of the two rollers is heated and the two strips are secured by calendering.
